(19) Europäisches Patentamt
European Patent Office
Office européen des brevets

(11) **EP 1 891 905 B1**

(12) **EUROPEAN PATENT SPECIFICATION**

(45) Date of publication and mention
of the grant of the patent:
**03.10.2018 Bulletin 2018/40**

(51) Int Cl.:
**A61B 17/32** *(2006.01)*

(21) Application number: **06756433.6**

(86) International application number:
**PCT/JP2006/310127**

(22) Date of filing: **22.05.2006**

(87) International publication number:
**WO 2006/134751 (21.12.2006 Gazette 2006/51)**

(54) **ULTRASONIC TREATMENT DEVICE, PROBE FOR ULTRASONIC TREATMENT DEVICE, AND FABRICATION METHOD THEREOF**

ULTRASCHALLBEHANDLUNGSGERÄT, SONDE FÜR EIN ULTRASCHALLBEHANDLUNGSGERÄT UND VERFAHREN ZU DEREN HERSTELLUNG

DISPOSITIF DE TRAITEMENT ULTRASONIQUE, SONDE POUR DISPOSITIF DE TRAITEMENT ULTRASONIQUE, ET PROCEDE DE FABRICATION IDOINE

(84) Designated Contracting States:
**DE FR GB**

(30) Priority: **16.06.2005 JP 2005176554**

(43) Date of publication of application:
**27.02.2008 Bulletin 2008/09**

(73) Proprietor: **Olympus Corporation**
**Tokyo 192-8507 (JP)**

(72) Inventor: **SAKAI, Ryoji**
**c/o IP Support Department**
**Hachioji-shi, Tokyo 192-8512 (JP)**

(74) Representative: **Thum, Bernhard**
**Wuesthoff & Wuesthoff**
**Patentanwälte PartG mbB**
**Schweigerstraße 2**
**81541 München (DE)**

(56) References cited:
**JP-A- 1 146 539      JP-A- 01 146 539**
**JP-A- 06 278 680      JP-A- 2002 035 001**
**JP-A- 2004 321 606    US-B1- 6 790 216**

EP 1 891 905 B1

## Description

Technical Field

This invention relates to an ultrasonic treatment apparatus that performs treatment on a living tissue by using ultrasonic waves, such as an ultrasonic coagulation and incision apparatus and an ultrasonic aspiration apparatus.

Background Art

In prior art, ultrasonic treatment apparatuses that perform treatment on a living tissue by using ultrasonic waves have been used. For example, Jpn. Pat. Appln. KOKAI Pub. No. 2004-321606 discloses an ultrasonic coagulation and incision apparatus that coagulates and incises a living tissue. The ultrasonic coagulation and incision apparatus of Jpn. Pat. Appln. KOKAI Pub. No. 2004-321606 has an ultrasonic oscillator that generates ultrasonic oscillations. The ultrasonic oscillator is connected with a proximal end portion of an elongated probe that transmits ultrasonic oscillations, and a distal end portion of the probe is provided with a treatment portion that performs coagulation and incision of a living tissue by the transmitted ultrasonic oscillations. The treatment portion projects from a tip opening of a sheath covering the probe, and a distal end of the sheath is provided with a jaw that is opened and closed with respect to the treatment portion and holds a tissue in cooperation with the treatment portion. When a living tissue is treated by the ultrasonic coagulation and incision apparatus, the treatment portion and the jaw hold the tissue therebetween, ultrasonic oscillations generated by the ultrasonic oscillator are transmitted to the treatment portion through the probe, and the treatment portion coagulates and incises the held tissue.

If a living tissue is treated in the state where the treatment portion is immersed in liquid such as humor and blood, the tissue may be damaged due to cavitation occurring on the treatment portion. USP 6,790,216 discloses an ultrasonic coagulation and incision apparatus that suppresses occurrence of cavitation on a treatment portion. The ultrasonic coagulation and incision apparatus of USP 6,790,216 has almost the same structure as that of the ultrasonic coagulation and incision apparatus of Jpn. Pat. Appln. KOKAI Pub. No. 2004-321606, and also has a structure wherein an inclined portion that is inclined toward the tip is provided with the treatment portion on the opposite side of its holding surface facing the jaw. USP 6,790,216 also discloses that reducing the inclination angle of the inclined portion suppresses cavitation occurring in the treatment portion.

On the other hand, Jpn. Pat. Appln. KOKAI Pub. No. 2002-233533 discloses an ultrasonic aspiration apparatus that crushes and aspirates a living tissue. The ultrasonic aspiration apparatus of Jpn. Pat. Appln. KOKAI Pub. No. 2002-233533 has an ultrasonic oscillator, a probe, and a sheath, which are similar to those of the ultrasonic coagulation and incision apparatuses disclosed in Jpn. Pat. Appln. KOKAI Pub. No. 2004-321606 and USP 6,790,216. Further, a treatment portion that emulsifies and crushes a living tissue is formed on a tip portion of the probe of the ultrasonic aspiration apparatus disclosed in Jpn. Pat. Appln. KOKAI Pub. No. 2002-233533. Further, an aspiration channel that has an opening in the treatment portion and aspirates the crushed tissue is formed between the probe and the sheath. When a living tissue is treated by the ultrasonic aspiration apparatus, ultrasonic oscillations generated by the ultrasonic oscillator are transmitted to the treatment portion through the probe, the treatment portion emulsifies and crushes a living tissue, and the crushed tissue is aspirated through the aspiration channel.

Disclosure of Invention

As described above, when a living tissue is coagulated and incised, the cavitation in the treatment portion should desirably be suppressed so as to prevent damage to the living tissue. On the other hand, when a living tissue is crushed and aspirated, the cavitation should desirably be promoted so as to effectively crush the living tissue.

USP 6,790,216 discloses suppressing the cavitation by reducing the inclination angle of an inclined portion that faces the holding surface of a treatment portion. However, USP 6,790,216 does not produce a cavitation state that is optimal to the treatment of a living tissue.

The present invention has been made in consideration of the above problems, and is intended to provide an ultrasonic treatment apparatus that enables a cavitation state optimal to the treatment of a living tissue. Accordingly, the above problems are obviated by a probe for an ultrasonic treatment apparatus according to claim 1 and a method of manufacturing such a probe according to claim 5. Preferred embodiments are given by the dependent claims. In an aspect of the present invention, a probe for an ultrasonic treatment apparatus is connected to an ultrasonic oscillator configured to generate longitudinal ultrasonic oscillations in a longitudinal direction of the probe, the probe is configured to transmit the ultrasonic oscillations generated by the ultrasonic oscillator, and the probe includes a treatment portion formed on the probe and is configured to treat a living tissue by the transmitted ultrasonic oscillations, the treatment portion includes a cavitation suppressing portion, and the treatment portion has a shape close to a streamline shape model wherein the streamline shape model has the pressures in the pressure distribution of a liquid greater than the saturation vapor pressure of the liquid when the ultrasonic oscillations are transmitted to the treatment portion from the ultrasonic oscillator in the liquid with a predetermined amplitude and period.

This ultrasonic treatment apparatus is featured in that when the treatment portion treats a living tissue in a liquid, the pressure of the liquid located in the vicinity of the external surface of the cavitation suppressing portion becomes greater than the saturation vapor pressure of the liquid. Accordingly, the cavitation in the treatment portion is suppressed. In an example, not according to the present invention, an ultrasonic treatment apparatus has the cavitation suppressing portion formed to have a shape having a small drag coefficient.

This ultrasonic treatment apparatus is featured in that when the treatment portion treats a living tissue in a liquid, the pressure of the liquid located in the vicinity of the external surface of the cavitation suppressing portion changes with a gentle slope and tends to be greater than the saturation vapor pressure of the liquid.

In a preferred aspect of the present invention, an ultrasonic treatment apparatus is characterized by used to coagulate and incise the living tissue.

This ultrasonic treatment apparatus is featured in that the treatment portion performs coagulation and incision in the state where the cavitation is suppressed in the treatment portion.

In a preferred aspect of the present invention, an ultrasonic treatment apparatus is characterized by further comprising: a jaw which is opened and closed with respect to the treatment portion, and holds the tissue in cooperation with the treatment portion.

This ultrasonic treatment apparatus is featured in that a living tissue is treated by the treatment portion in the state where it is held by the treatment portion and jaw.

In another aspect of the present invention, an ultrasonic treatment apparatus characterized by further comprising: an ultrasonic oscillator which generates ultrasonic waves.

This ultrasonic treatment apparatus is featured in that when the treatment portion treats a living tissue in a liquid, the pressure of the liquid located in the vicinity of the external surface of the cavitation promoting portion becomes equal to or less than the saturation vapor pressure of the liquid. Accordingly, the cavitation in the treatment portion is promoted. In an example, not according to the present invention, an ultrasonic treatment apparatus has the cavitation promoting portion formed to have a shape having a large drag coefficient.

This ultrasonic treatment apparatus is featured in that when the treatment portion treats a living tissue in a liquid, the pressure of the liquid located in the vicinity of the external surface of the cavitation suppressing portion changes with a steep slope and tends to be equal to or less than the saturation vapor pressure of the liquid. In an example, not according to the present invention, an ultrasonic treatment apparatus is used to crush and aspirate the living tissue and further comprises an aspiration channel which aspirates the crushed living tissue.

This ultrasonic treatment apparatus is featured in that the treatment portion crushes a living tissue in the state where the cavitation is promoted in the treatment portion, and the crushed living tissue is aspirated into the aspiration channel. In an example, not according to the present invention, an ultrasonic treatment apparatus has the treatment portion formed to have a shape such that a direction of a velocity of the liquid in the vicinity of the external surface of the cavitation promoting portion corresponds to a direction from the treatment portion toward the tissue in treatment on the tissue in the fluid analysis concerning the ultrasonic oscillations in the liquid.

The ultrasonic treatment apparatus is featured in that when the treatment portion treats a living tissue in a liquid, the cavitation occurring in the vicinity of the external surface of the cavitation promoting portion moves toward the living tissue. In an example, not according to the present invention, a probe for an ultrasonic treatment apparatus is used in the above ultrasonic treatment apparatus.

In another aspect of the present invention, a method of manufacturing a probe for an ultrasonic treatment apparatus is characterized by comprising: preparing a predetermined shape model for at least part of a treatment portion which treats a living tissue by ultrasonic oscillations; obtaining, by fluid analysis concerning ultrasonic oscillations in a liquid, a pressure distribution of the liquid with respect to the shape model; changing a shape of an edge portion of the shape model to a shape close to a streamline shape such that the pressure of at least part of portions where the pressure is equal to or less than a saturation vapor pressure of the liquid in the pressure distribution becomes greater than the saturation vapor pressure of the liquid; alternately repeating the obtaining the pressure distribution of the liquid and the changing the shape of the shape model; and forming the treatment portion to have the shape close to a streamline shape of the shape model after ending the changing of the shape close to a streamline shape of the edge portion of the shape model when the pressure of the at least part of the portions becomes greater than the saturation vapor pressure of the liquid.

In a preferred aspect of the present invention, a method of manufacturing a probe for an ultrasonic treatment apparatus is characterized in that the changing the shape of the shape model includes changing the shape of the shape model such that a drag coefficient is reduced. In an example, not according to the present invention, a method of manufacturing a probe for an ultrasonic treatment apparatus comprises: preparing a predetermined shape model for at least part of a treatment portion which treats a living tissue by ultrasonic oscillations; obtaining, by fluid analysis concerning ultrasonic oscillations in a liquid, a pressure distribution of the liquid with respect to the shape model; changing a shape of the shape model such that a pressure of at least part of portions where the pressure is greater than a saturation vapor pressure of the liquid in the pressure distribution becomes less than the saturation vapor pressure of the liquid; alternately repeating the obtaining the pressure distribution of the liquid and the changing the shape of the shape model; and forming the treatment portion to have a shape of the shape model.

In a preferred aspect of the present invention, a method of manufacturing a probe for an ultrasonic treatment apparatus is characterized in that the changing the shape of the shape model includes changing the shape of the shape model such that a drag coefficient is increased. In an example, not according to the present invention, a method of manufacturing a probe for an ultrasonic treatment apparatus further comprises: changing the shape of the shape model such that a direction of a velocity of the liquid in at least part of portions where the pressure is less than the saturation vapor pressure of the liquid in the pressure distribution corresponds to a direction from the treatment portion toward the tissue in treatment on the tissue.

In another aspect of the present invention, a method of manufacturing an ultrasonic treatment apparatus is characterized by comprising the above method of manufacturing a probe for an ultrasonic treatment apparatus.

The ultrasonic treatment apparatus according to the present invention enables a cavitation state that is optimal to the treatment of a living tissue.

In addition, the method of manufacturing a probe for use in an ultrasonic treatment apparatus, according to the present invention, enables the manufacture of an ultrasonic treatment apparatus capable of producing a cavitation state that is optimal to the treatment of a living tissue.

Brief Description of Drawings

FIG. 1 is a side view of an ultrasonic coagulation and incision apparatus.

FIG. 2 is a perspective view of a tip portion of the ultrasonic coagulation and incision apparatus according to the first embodiment of the present invention.

FIG. 3A is a perspective view of a probe of the ultrasonic coagulation and incision apparatus in an oscillation state toward a tip side.

FIG. 3B is a perspective view of the probe of the ultrasonic coagulation and incision apparatus in an oscillation state toward a rear end side.

FIG. 4 is a perspective view of an initial three-dimensional model of a treatment portion, in a method of designing the treatment portion of the ultrasonic coagulation and incision apparatus according to the first embodiment of the present invention.

FIG. 5 is a pressure distribution diagram for the initial three-dimensional model prepared as a result of fluid analysis, in the method of designing the treatment portion of the ultrasonic coagulation and incision apparatus according to the first embodiment of the present invention.

FIG. 6 is a velocity distribution diagram for the initial three-dimensional model prepared as a result of fluid analysis, in the method of designing the treatment portion of the ultrasonic coagulation and incision apparatus according to the first embodiment of the present invention.

FIG. 7 is a perspective view of a final three-dimensional model of the treatment portion, in the method of designing the treatment portion of the ultrasonic coagulation and incision apparatus according to the first embodiment of the present invention.

FIG. 8 is a pressure distribution diagram of the final three-dimensional model prepared as a result of fluid analysis, in the method of designing the treatment portion of the ultrasonic coagulation and incision apparatus according to the first embodiment of the present invention.

FIG. 9 is a perspective view of a probe of an ultrasonic coagulation and incision apparatus according to a modification of the first embodiment of the present invention, in an oscillation state.

FIG. 10 is a side view of an ultrasonic aspiration apparatus.

FIG. 11 is a perspective view of an initial three-dimensional model of a treatment portion, in a method of designing the treatment portion of the ultrasonic coagulation and incision apparatus.

FIG. 12 is a pressure distribution diagram for the initial three-dimensional model prepared as a result of fluid analysis, in the method of designing the treatment portion of the ultrasonic coagulation and incision apparatus.

FIG. 13 is a perspective view of a final three-dimensional model of the treatment portion, in the method of designing the treatment portion of the ultrasonic coagulation and incision apparatus.

FIG. 14 is a pressure distribution diagram for the initial three-dimensional model prepared as a result of fluid analysis, in the method of designing the treatment portion of the ultrasonic coagulation and incision apparatus.

FIG. 15 is a diagram illustrating values of a drag coefficient $C_D$ with respect to Reynolds numbers Re for various forms.

Best Mode for Carrying Out the Invention

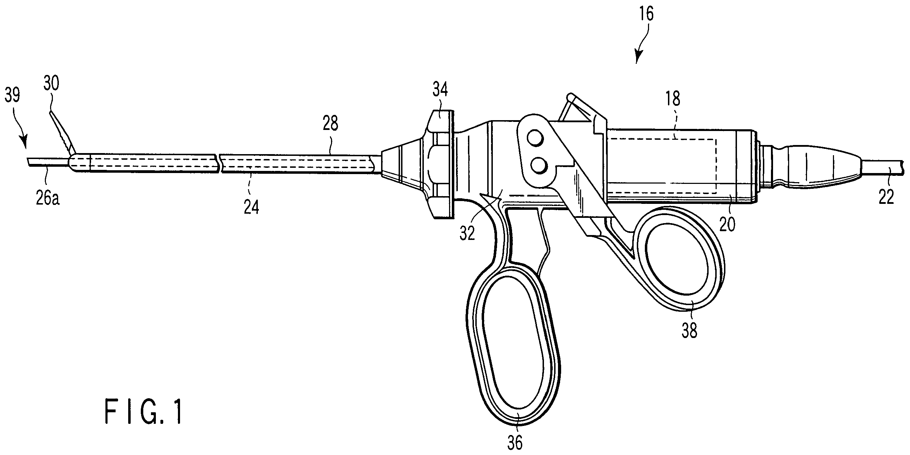

A first embodiment of the present invention is described with reference to FIGS. 1 to 8. The ultrasonic treatment apparatus according to the first embodiment is an ultrasonic coagulation and incision apparatus 16 that suppresses occurrence of cavitation. As shown in FIG. 1, the ultrasonic coagulation and incision apparatus 16 has an ultrasonic oscillator 18 that generates ultrasonic oscillations. The ultrasonic oscillator 18 is accommodated in a cylindrical cover 20. A code 22 to supply electric power to the ultrasonic oscillator 18 is extended out of a proximal end portion of the cylindrical cover 20.

Further, an output end on a tip portion of the ultrasonic oscillator 18 is connected with a proximal end portion of a probe 24 transmitting the ultrasonic oscillations and having an elongated straight shape. A treatment portion 26a that coagulates and incises a living tissue by the transmitted ultrasonic oscillations is formed on a tip portion of the probe 24.

Further, the probe 24 is covered with a sheath 28. A jaw 30 that is opened and closed with respect to the treatment portion 26a and holds a living tissue in cooperation with the treatment portion 26a is provided on a tip portion of the sheath 28. On the other hand, a proximal end portion of the sheath 28 is connected with an operation main body 32 such that the sheath 28 is rotatable around its central axis. A rotary knob 34 to rotate the sheath 28 is provided on the proximal end portion of the sheath 28. Further, a fixed handle 36 and a movable handle 38 to open and close the jaw 30 are provided on the operation main body 32. Specifically, the movable handle 38 is pivotally supported by the operation main body 32 such that the movable handle 38 is openable and closable with respect to the fixed handle 36, and is pivotally supported by a proximal end portion of an operation rod located in the operation main body 32. The operation rod is inserted through the operation main body 32 and the sheath 28 such that the operation rod is movable back and forth, and the tip portion of the operation rod is connected to a proximal end of the jaw 30. Further, the operation rod is moved back and forth by opening and closing the movable handle 38 with respect to the fixed handle 36, and thereby the jaw 30 is opened and closed with respect to the treatment portion 26a.

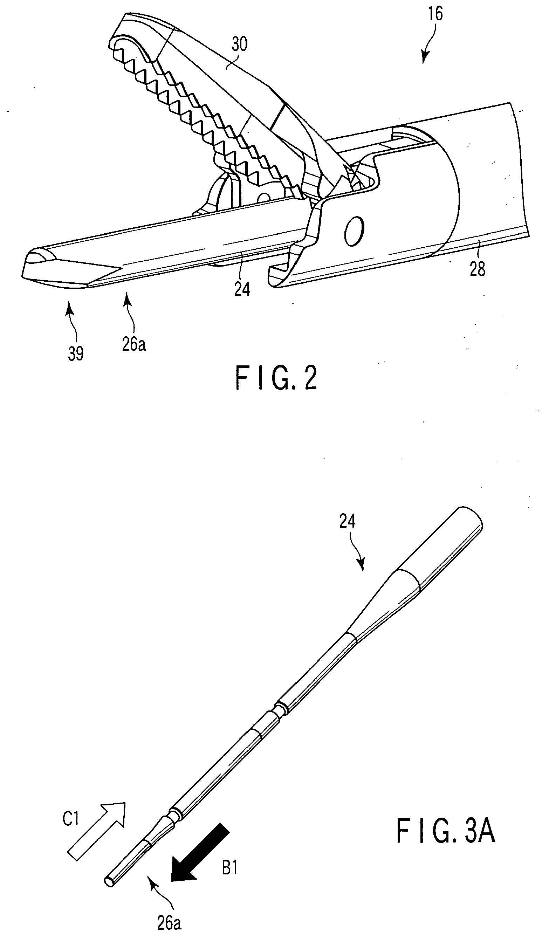

The treatment portion 26a illustrated in FIG. 2 according to the first embodiment has a form that suppresses generation of cavitation in the case where it is oscillated by ultrasonic waves in liquid such as humor and blood. The following is explanation of a method of designing the treatment portion 26a.

Step 1: Preparation of initial three-dimensional model

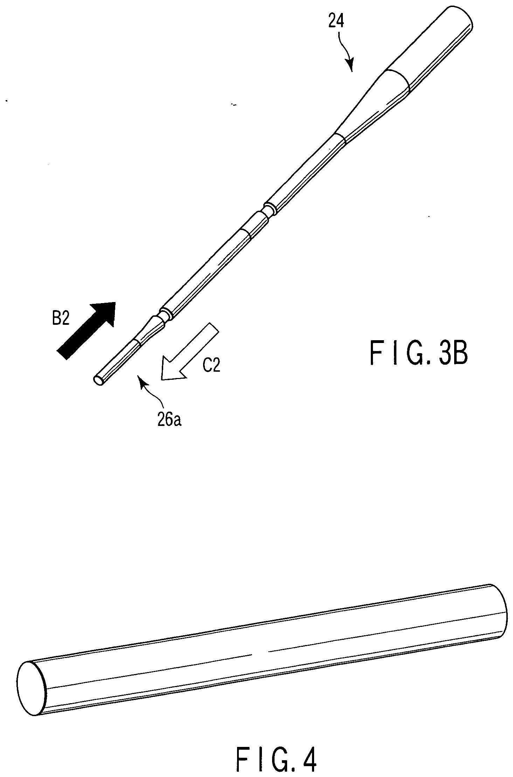

An initial three-dimensional model is prepared for the probe 24 as shown in FIGS. 3A and 3B. In the first embodiment, a conventional ultrasonic coagulation and incision probe is adopted as the initial three-dimensional model.

Step 2: Fluid analysis based on the three-dimensional model

Fluid analysis is performed for the case where the probe 24 is oscillated in liquid by ultrasonic waves.

The probe 24 is longitudinally oscillated in liquid in its longitudinal direction with a predetermined amplitude and period. Specifically, the probe 24 repeats oscillation toward the tip side illustrated by arrow B1 in FIG. 3A, and oscillation toward the proximal end side shown by arrow B2 in FIG. 3B. In the first embodiment, analysis is performed by using a coordinate system fixed on the probe 24. In the coordinate system, the probe 24 is located in a state of rest in a liquid field that oscillates with a predetermined amplitude and period, such that the longitudinal direction of the probe 24 coincides with the oscillation direction of the liquid. Specifically, the liquid repeats oscillation toward the proximal end side illustrated by arrow C1 in FIG. 3A, and oscillation toward the tip side illustrated by arrow C2 in FIG. 3B.

In the first embodiment, fluid analysis is performed only with respect to the treatment portion 26a in the tip portion of the probe 24, to reduce analysis time in the fluid analysis. Specifically, a three-dimensional model of the treatment portion 26a whose both end portions have the same shape as those of the treatment portion 26a is prepared on the basis of the three-dimensional model of the probe 24. FIG. 4 illustrates an example of the prepared three-dimensional model of the treatment portion 26a. The three-dimensional model of the treatment portion 26a corresponds to a cylindrical shape adopted in the treatment portion 26a of a conventional ultrasonic coagulation and incision probe 24.

Then, prepared is a liquid field model for a half period of the above liquid field that oscillates in the one direction with a predetermined amplitude and period, that is, a liquid field model whose amplitude increases from 0 to its maximum amplitude and decreases from the maximum amplitude to 0 with a predetermined period, and then amplitude increases again without decrease. The three-dimensional model of the treatment portion 26a is located in a state of rest in the liquid field model such that the longitudinal direction of the three-dimensional model coincides with the oscillation direction of the liquid, and then fluid analysis is performed. In the three-dimensional model of the treatment portion 26a, at an end portion on an upstream side of the oscillation direction of the liquid field model, behavior when the treatment portion 26a is oscillated toward the tip side is analyzed. At an end portion on a downstream side of the oscillation direction, behavior when the treatment portion 26a is oscillated toward the proximal end side is analyzed. In fluid analysis, a pressure distribution and a velocity distribution of the liquid field model are calculated.

Based on the pressure distribution of the liquid field model, occurrence of cavitation is analyzed. Generally, cavitation occurs if the liquid reaches its saturation vapor pressure. For example, water reaches its saturation vapor pressure and cavitation occurs, when its temperature is increased to 100°C under atmospheric pressure (101.3 kPa), and when its pressure is reduced to 2 kPa under standard temperature (20°C). If a living tissue is treated in liquid with the treatment portion 26a, it is expected that cavitation occurs in portions corresponding to portions of the liquid, whose pressures are reduced to its saturation vapor pressure in the liquid field model.

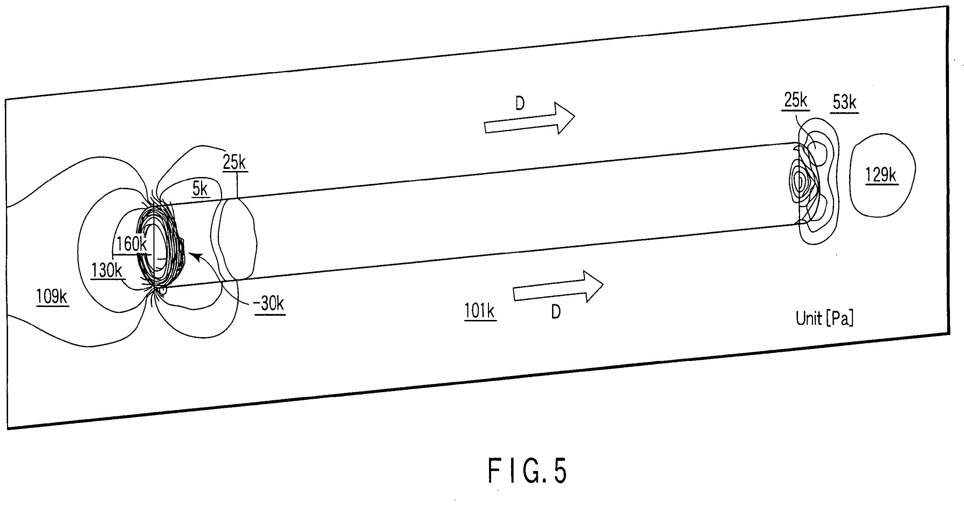

FIG. 5 illustrates an example of a pressure distribution diagram prepared as a result of the fluid analysis. In FIG. 5, the oscillation direction of the liquid is indicated by arrows D. Water at standard temperature (20°C) is selected as the liquid of the liquid field model. It is expected that cavitation occurs in portions where the pressure is equal to or less than the saturation vapor pressure (2 kPa) in the liquid field model. As shown in FIG. 5, the pressure of the liquid field model is 2 kPa or less in the vicinity of an edge of the upstream end portion in the three-dimensional model of the treatment portion 26a. Thus, if a living tissue is treated in liquid with the treatment portion 26a, it is expected that cavitation occurs in the vicinity of an edge portion of the treatment portion 26a when the treatment portion 26a is oscillated toward the tip side. In a corresponding actual experiment, it has been verified that cavitation occurs in an edge portion of the treatment portion 26a when it is oscillated toward the tip side if a living tissue is treated in liquid with the treatment portion 26a.

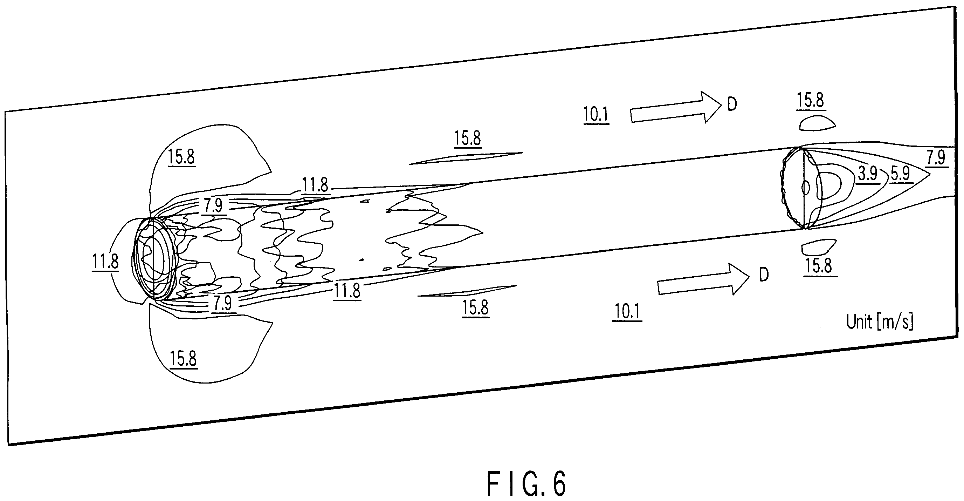

FIG. 6 illustrates an example of a velocity distribution diagram prepared as a result of the fluid analysis. In FIG. 6, the oscillation direction of the liquid is indicated by arrows D. As shown in FIG. 6, it is understood that the velocity of the liquid of the liquid field model converges on one point in a downstream end portion of the three-dimensional model of the treatment portion 26a. Specifically, cavitation generated in an edge portion of the treatment portion 26a when the treatment portion 26a is oscillated toward the tip side is assumed to move from the edge portion of the treatment portion 26a toward the tip side when the treatment portion 26a is oscillated toward the proximal end side. Also in a corresponding actual experiment, it has been verified that cavitation moves from the edge portion of the treatment portion 26a toward the tip side in oscillation of the treatment portion 26a toward the proximal end side.

Step 3: Change in shape of the three-dimensional model

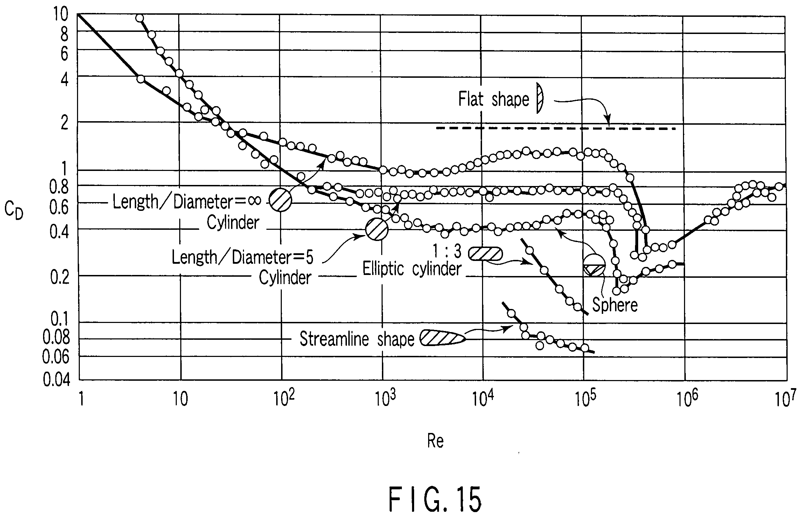

The shape of the three-dimensional model of the treatment portion 26a is changed such that the pressures of portions where the pressure is equal to or less than the saturation vapor pressure of the liquid become greater than the saturation vapor pressure. In the first embodiment, the shape of portions of the three-dimensional model in the vicinity of portions of the liquid field model, where the pressure is equal to or less than the saturation vapor pressure of the liquid, is changed to a shape having a smaller drag coefficient. If the drag coefficient is small, a pressure gradient becomes gentler, and decrease in the pressure of the liquid in the liquid field model is suppressed. Specifically, in FIG. 5, the pressure of the liquid field model in the vicinity of the edge portion of the upstream end portion in the three-dimensional model of the treatment portion 26a is equal to or less than the saturation vapor pressure (2 kPa). The shape of the edge portion is changed to a streamline shape having a small drag coefficient. As a matter of course, the shape of the edge portion of the downstream end is also changed in conformity with the modification of the edge portion of the upstream end. FIG. 15 illustrates values of drag coefficient $C_D$ for Reynolds numbers Re for various shapes.

Step 4: Repetition of fluid analysis based on the three-dimensional model and change in shape of the three-dimensional model

The fluid analysis based on the three-dimensional model of Step 2 and the change in shape of the three-dimensional model of Step 3 are alternately repeated.

Step 5: Determination of final three-dimensional model

When the portions in the liquid field model where the pressure is reduced to the saturation vapor pressure of the liquid are almost disappeared, change in shape of the three-dimensional model is ended, and the final three-dimensional model of the treatment portion 26a is determined.

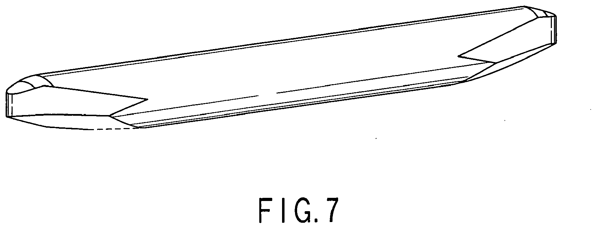

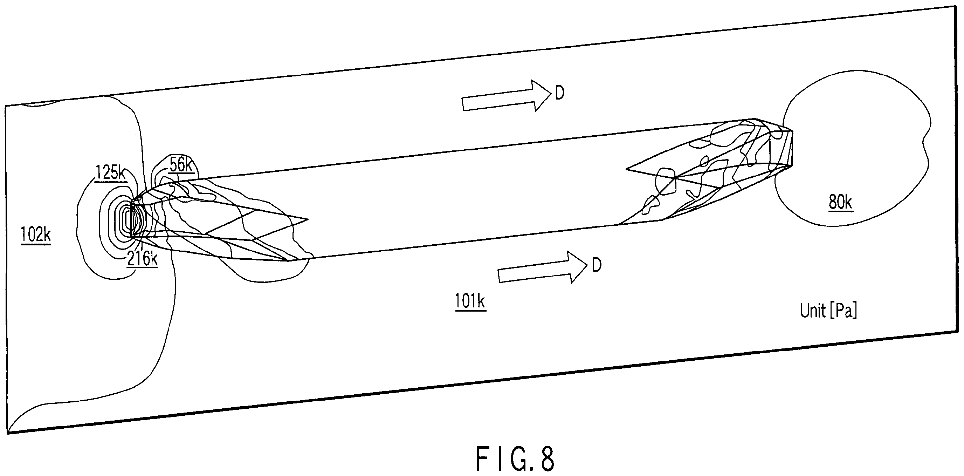

FIG. 7 illustrates an example of the final three-dimensional model of the treatment portion 26a. As shown in FIG. 7, the three-dimensional model of the treatment portion 26a has a shape close to a streamline shape. FIG. 8 is a pressure distribution diagram prepared as a result of the fluid analysis of the three-dimensional model of the treatment portion 26a. In FIG. 8, the oscillation direction of the liquid is indicated by arrows D. As shown in FIG. 8, the portions where the pressure is equal to or less than the saturation vapor pressure (2 kPa) have almost been disappeared. Therefore, it is expected that occurrence of cavitation is suppressed in the case where a living tissue is treated in liquid with the treatment portion 26a. Also in a corresponding actual experiment, it has been verified that occurrence of cavitation is suppressed if a living tissue is treated in liquid with the treatment portion 26a.

As described above, according to the first embodiment, the tip portion of the treatment portion 26a is a cavitation suppressing portion 39 that suppresses occurrence of cavitation.

Next, operation of the ultrasonic coagulation and incision apparatus 16 according to a preferred embodiment is explained. When a living tissue is treated with the ultrasonic coagulation and incision apparatus 16, the treatment portion 26a and the jaw 30 hold the tissue therebetween, ultrasonic oscillations generated by the ultrasonic oscillator 18 are transmitted to the treatment portion 26a through the probe 24, and then the treatment portion 26a coagulates and incises the held tissue. In this process, although the treatment portion 26a may be immersed in liquid such as humor and blood, the pressure gradient of the liquid is gentle in the vicinity of the external surface of the treatment portion 26a, and thus the pressure of the liquid rarely becomes equal to or less than the saturation vapor pressure of the liquid. Therefore, occurrence of cavitation in the treatment portion 26a is suppressed.

Therefore, the ultrasonic coagulation and incision apparatus 16 of the first embodiment has the following effects. The treatment portion 26a of the first embodiment is formed to have a shape such that the pressure in the vicinity of the external surface of the treatment portion 26a is greater than the saturation vapor pressure of the liquid, in the fluid analysis concerning the ultrasonic oscillation in the liquid. Further, occurrence of cavitation in the treatment portion 26a when the treatment portion 26a coagulates and incises a living tissue in liquid is actually suppressed, and an optimum cavitation state in coagulation and incision is realized.

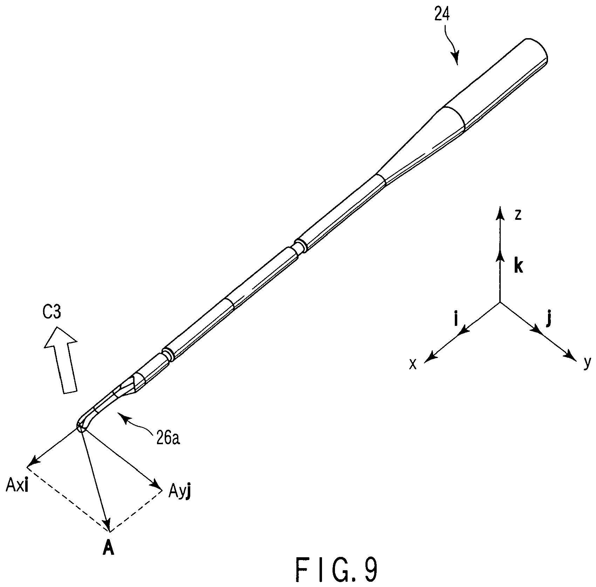

A modification in an example, not according to the present invention is explained with reference to FIG. 9. In the modification, an optimum cavitation state is realized for the treatment portion 26a that is oscillated in a three-dimensional manner.

In the probe 24 having a straight shape as in the first embodiment, the treatment portion 26a is oscillated in a one-dimensional manner. On the other hand, in a common probe 24, the treatment portion 26a is oscillated in a three-dimensional manner. Specifically, an amplitude vector of the treatment portion 26a is represented as follows by using vector components in X, Y and Z axis directions.

[Math 1]

$$A = Ax \cdot i + Ay \cdot j + Az \cdot k$$

i, j, k: unit vectors of respective axis directions
Ax, Ay, Az: sizes of amplitudes of respective axis directions

The sizes of amplitudes of the respective axis directions can be calculated by numerical analysis. A liquid field model used for fluid analysis in design of the treatment portion 26a is prepared on the basis of the sizes of the amplitudes of the respective axis directions.

For example, in the probe 24 having a curved shape as shown in FIG. 9, the treatment portion 26a is oscillated in a two-dimensional manner. In this case, sizes of amplitudes of the X axis and the Y axis are calculated by numerical analysis, and the liquid field model used for the fluid analysis in the method of designing the treatment portion 26a is prepared as indicated by arrow C3.

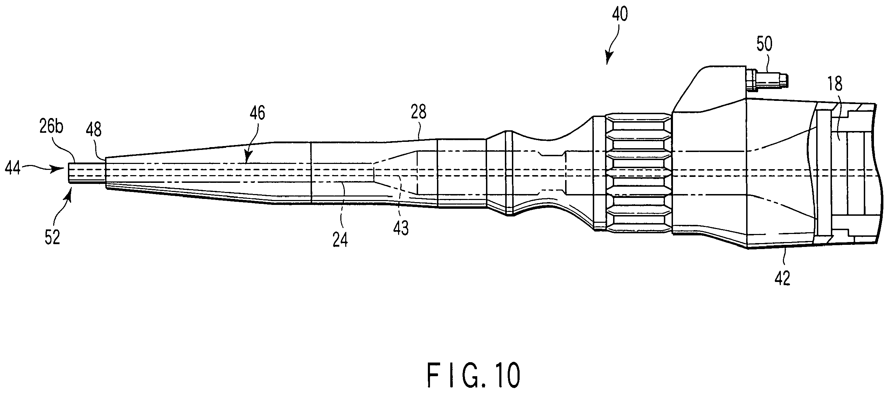

FIGS. 10 to 14 illustrate further example, not according to the present invention. Structures having similar functions as those in the first embodiment are denoted by the same reference numerals as those in the first embodiment, and explanation thereof is omitted. The ultrasonic treatment apparatus in this example, not according to the present invention, is an ultrasonic aspiration apparatus 40 that crushes and aspirates a living tissue. As shown in FIG. 10, an ultrasonic oscillator 18 of the ultrasonic aspiration apparatus 40 is accommodated in a hand piece 42. Further, an output end of the ultrasonic oscillator 18 is connected with a proximal end portion of a probe 24, and a treatment portion 26b that emulsifies and crushes a living tissue by transmitted ultrasonic oscillations is formed on a tip portion of the probe 24.

Further, an aspiration channel 43 to aspirate the crushed tissue is formed through the probe 24 and the ultrasonic oscillator 18 in the longitudinal direction of the probe 24 and the ultrasonic oscillator 18. A tip portion of the aspiration channel 43 is opened at the treatment portion 26b and forms an aspiration opening portion 44. A proximal end portion of the aspiration channel 43 communicates with an aspiration connecter formed in the hand piece 42, and the aspiration connecter is connected to an aspiration apparatus.

Further, the probe 24 is covered with a sheath 28, and a clearance between the probe 24 and the sheath 28 forms a liquid conveying channel 46 to convey liquid. A tip portion of the liquid conveying channel 46 is annularly opened between the tip portion of the sheath 28 and the probe 24 and forms a liquid conveying opening portion 48. A proximal end portion of the liquid conveying channel 46 communicates with a liquid conveying connecter 50 provided on the hand piece, and the liquid conveying connecter 50 is connected with a liquid conveying apparatus.

The treatment portion 26b in this example, not according to the present invention, has a shape that promotes occurrence of cavitation when the treatment portion 26b is oscillated by ultrasonic waves in liquid such as a physiological saline solution. A method of designing the treatment portion 26b is explained below. Explanation of steps similar to those in the designing method according to the first embodiment is omitted.

Step 1: Preparation of initial three-dimensional model

In this example, not according to the invention, a conventional ultrasonic aspiration probe is adopted as an initial three-dimensional model.

Step 2: Fluid analysis based on the three-dimensional model

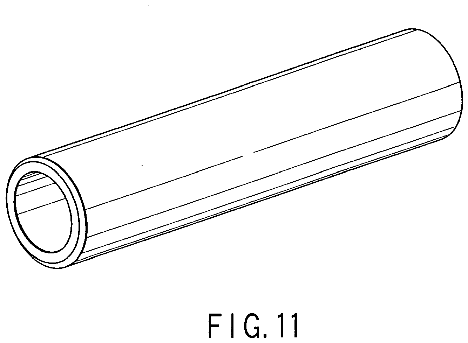

As shown in FIG. 11, an almost cylindrical three-dimensional model of the treatment portion 26b is prepared on the basis of a three-dimensional model of the probe 24. Both ends of the three-dimensional model of the treatment portion 26b have the same shape as those of the treatment portion 26b.

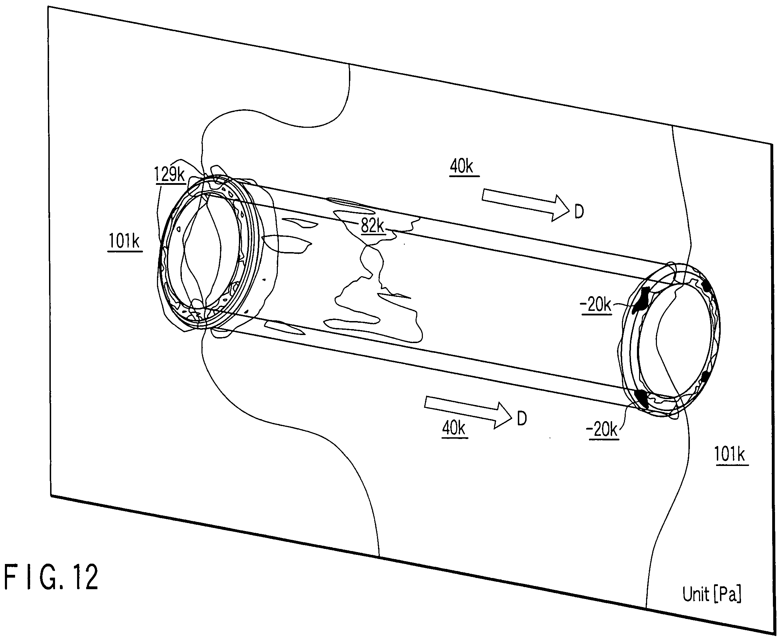

FIG. 12 illustrates an example of a pressure distribution diagram prepared as a result of fluid analysis. In FIG. 12, the oscillation direction of the liquid is indicated by arrows D. As shown in FIG. 12, the pressure of the liquid field model is 2 kPa or less in the vicinity of an annular end surface of a downstream end portion in the three-dimensional model of the treatment portion 26b.

Therefore, if a living tissue is actually treated in liquid with the treatment portion 26b, it is expected that cavitation occurs in the vicinity of the annular end surface of the treatment portion 26b when the treatment portion 26b is oscillated toward the rear end side.

Step 3: Change in shape of the three-dimensional model

The shape of the three-dimensional model of the treatment portion 26b is changed such that, with respect to portions where cavitation is required to occur when a living tissue is treated in liquid with the treatment portion 26b, the pressure in corresponding portions in the liquid field model is equal to or less than the saturation vapor pressure.

In this example, not according to the present invention, the shape of the three-dimensional model in the vicinity of portions of the liquid field model where the pressure is required to be equal to or lower than the saturation vapor pressure of the liquid is changed to a shape having a large drag coefficient. Increasing a drag coefficient makes a pressure gradient steep, and increases reduction in pressure of the liquid in the liquid field model. Specifically, with reference to FIG. 12, if the pressure of the portions of the liquid field model in the vicinity of the annular end surfaces in the both end portions of the three-dimensional model of the treatment portion 26b is required to be equal to or lower than the saturation vapor pressure (2 kPa), the shape of the three-dimensional model is changed such that the both end portions of the external peripheral portions in the three-dimensional model of the treatment portion 26b have the same flange shape to increase the drag coefficient with respect to the oscillation direction of the liquid field.

Step 4: Repetition of fluid analysis based on the three-dimensional model and the change in shape of the three-dimensional model

Step 5: Determination of final three-dimensional model

When the pressure in the portions in the liquid field model, which is required to be equal to or less than the saturation vapor pressure, has become equal to or less than the saturation vapor pressure, change in shape of the three-dimensional model is ended, and a final three-dimensional model of the treatment portion 26b is determined.

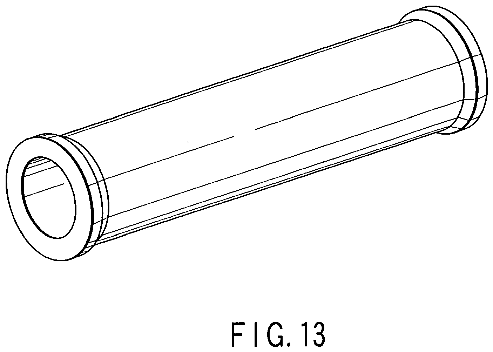

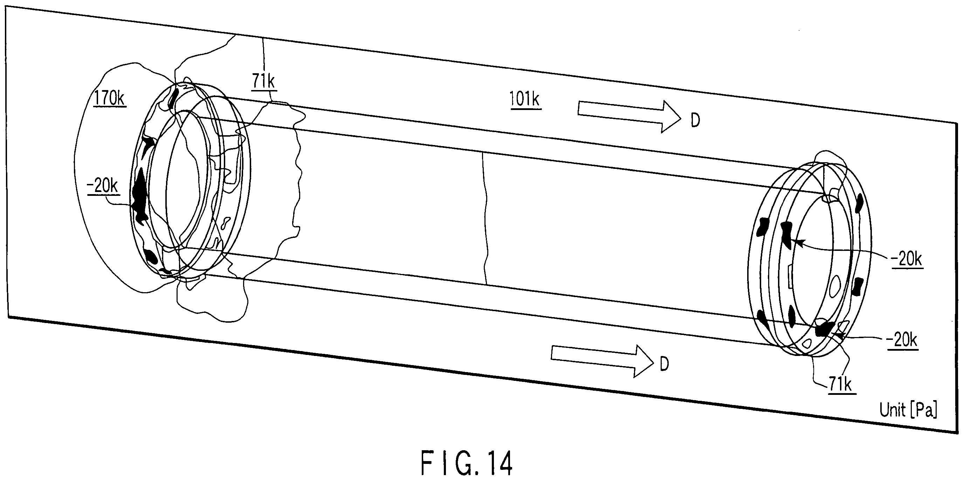

FIG. 13 illustrates an example of the final three-dimensional model of the treatment portion 26b. As shown in FIG. 13, the three-dimensional model of the treatment portion 26b has a shape in which the end portions have a flange shape. FIG. 14 is a pressure distribution diagram prepared as a result of the fluid analysis with respect to the three-dimensional model of the treatment portion 26b. In FIG. 14, the oscillation direction of the liquid is indicated by arrows D. As shown in FIG. 14, portions where the pressure is equal to or less than the saturation vapor pressure (2 kPa) are formed in the vicinity of the annular end surfaces in the both end portions of the three-dimensional model of the treatment portion 26b. Therefore, it is expected that occurrence of cavitation is promoted in the case where a living tissue is treated in liquid with the treatment portion 26b. Also in a corresponding actual experiment, it has been verified that occurrence of cavitation is promoted if a living tissue is treated in liquid with the treatment portion 26b.

As described above, according to this example, not according to the present invention, the tip portion of the treatment portion 26a is a cavitation promoting portion 52 that promotes occurrence of cavitation.

Next, operation of the ultrasonic aspiration apparatus 40 according to the example, not according to the present invention, is explained. When a living tissue is treated with the ultrasonic aspiration apparatus 40, an aspiration apparatus and a liquid conveying apparatus are connected to the aspiration connecter and the liquid conveying connecter 50, respectively. Then, the treatment portion 26b and the tissue are immersed in liquid such as physiological saline solution by conveying the liquid through the liquid conveying opening portion 48. In this state, ultrasonic oscillations generated by the ultrasonic oscillator 18 are transmitted to the treatment portion 26b through the probe 24, and the treatment portion 26b is pressed onto the tissue to emulsify and crush the tissue. In this process, the treatment portion 26b is immersed in liquid such as physiological saline solution, the pressure gradient of the liquid is steep in the vicinity of the external surface of the treatment portion 26b, and thus the pressure of the liquid becomes equal to or less than the saturation vapor pressure of the liquid. Therefore, occurrence of cavitation in the treatment portion 26b is promoted. Thus, emulsification and crushing of the tissue are effectively performed. The emulsified and crushed tissue is aspirated through the aspiration channel 43 via the aspiration opening portion 44.

Therefore, the ultrasonic aspiration apparatus 40 of this example, not according to the present invention has the following effects. The treatment portion 26b of the example is formed to have a shape such that the pressure in the vicinity of the external surface of the treatment portion 26b is equal to or less than the saturation vapor pressure of the liquid, in the fluid analysis concerning the ultrasonic oscillation in the liquid. Further, occurrence of cavitation in the treatment portion 26b when the treatment portion 26b emulsifies and crushes a living tissue in liquid is actually promoted, and an optimum cavitation state in emulsification and crushing is realized.

The following is explanation of a modification of the example not according to the present invention. The treatment portion 26b in the modification has a shape such that occurred cavitation moves toward the tissue when the treatment portion 26b is oscillated by ultrasonic waves in liquid such as physiological saline solution.

In a method of designing the treatment portion 26b of the modification, in a step of changing the shape of the three-dimensional model, the shape of the three-dimensional model of the treatment portion 26b is changed such that the direction of velocity of the liquid in portions of the liquid field model where the pressure is equal to or less than the saturation vapor pressure of the liquid corresponds to the direction from the treatment portion 26b toward the tissue in treatment on the tissue. Specifically, with reference to FIG. 14, the shape of the three-dimensional model is changed such that the direction of velocity of the liquid in the portions of the liquid field model, where the pressure is equal to or less than the saturation vapor pressure (2 kPa) of the liquid, in the vicinity of the end portions in the three-dimensional model of the treatment portion 26b corresponds to the direction from the treatment portion 26b toward the tissue in treatment on the tissue, that is, an outward longitudinal direction of the treatment portion 26b.

When a living tissue is treated with the ultrasonic aspiration apparatus 40 according to the modification, cavitation generated by the treatment portion 26b moves toward the tissue, and reaches the tissue to promote emulsification and crushing of the tissue. Therefore, according to the treatment portion 26b of the modification, cavitation generated by the treatment portion 26b efficiently reaches the tissue, and promotes emulsification and crushing of the tissue.

Industrial Applicability

The present invention provides an ultrasonic treatment apparatus that performs treatment on a living tissue by using ultrasonic waves, such as an ultrasonic coagulation and incision apparatus and an ultrasonic aspiration apparatus, and enables a cavitation state optimal to the treatment of a living tissue.

**Claims**

1. A probe (24) for an ultrasonic treatment apparatus, the probe being connected to an ultrasonic oscillator (18) configured to generate longitudinal ultrasonic oscillations in a longitudinal direction of the probe (24), the probe being configured to transmit the ultrasonic oscillations generated by the ultrasonic oscillator (18), and which includes a treatment portion (26a) formed on the probe (24) and configured to treat a living tissue by the transmitted ultrasonic oscillations, the treatment portion (26a) including a cavitation suppressing portion (39), and that the treatment portion (26a) has a shape close to a streamline shape model,
   **characterized by**
   the shape close to a streamline shape model having the pressures in a pressure distribution of a liquid greater than the saturation vapor pressure of the liquid when the ultrasonic oscillations are transmitted to the treatment portion from the ultrasonic oscillator in the liquid with a predetermined amplitude and period.

2. An ultrasonic treatment apparatus **characterized by** comprising the probe (24) according to claim 1, and the ultrasonic treatment apparatus is **characterized by** being used to coagulate and incise the living tissue.

3. An ultrasonic treatment apparatus **characterized by** comprising:

   the probe (24) according to claim 1; and
   a jaw (30) which is opened and closed with respect to the treatment portion (26a), and holds the tissue in cooperation with the treatment portion (26a).

4. An ultrasonic treatment apparatus **characterized by** comprising:

   an ultrasonic oscillator (18) which generates ultrasonic oscillations; and
   the probe (24) according to claim 1.

5. A method of manufacturing a probe for an ultrasonic treatment apparatus, comprising:

   preparing a predetermined shape model for at least part of a treatment portion which treats a living tissue by ultrasonic oscillations;
   the method further being **characterized by** obtaining, by fluid analysis concerning ultrasonic oscillations in a liquid, a pressure distribution of the liquid with respect to the shape model;
   changing a shape of an edge portion of the shape model to a shape close to a streamline shape such that the pressure of at least part of portions where the pressure is equal to or less than a saturation vapor pressure of the liquid in the pressure distribution becomes greater than the saturation vapor pressure of the liquid;
   alternately repeating the obtaining the pressure distribution of the liquid and the changing the shape of the edge portion of the shape model; and
   forming the treatment portion to have a shape close to a streamline shape of the shape model after ending the changing of shape close to a streamline shape of the edge portion of the shape model when the pressure of the at least part of the portions becomes greater than the saturation vapor pressure of the liquid.

6. The method according to claim 5, **characterized in that** the changing the shape of the shape model includes changing the shape of the shape model such that a drag coefficient is reduced.

**7.** A method of manufacturing an ultrasonic treatment apparatus, **characterized by** comprising the method of manufacturing a probe for an ultrasonic treatment apparatus according to claim 5 or 6.

**Patentansprüche**

**1.** Sonde (24) für eine Ultraschallbehandlungsvorrichtung, die Sonde ist mit einem Ultraschalloszillator (18) verbunden, welcher konfiguriert ist, um longitudinale Ultraschallschwingungen in einer longitudinalen Richtung der Sonde (24) zu erzeugen, die Sonde ist konfiguriert, um die von dem Ultraschalloszillator (18) erzeugten Ultraschallschwingungen zu übertragen, und beinhaltet einen auf der Sonde (24) gebildeten Behandlungsabschnitt (26a) und ist konfiguriert, um lebendes Gewebe mit den übertragenen Ultraschallschwingungen zu behandeln, der Behandlungsabschnitt (26a) beinhaltet einen Kavitationsunterdrückungsabschnitt (39) und der Behandlungsabschnitt (26a) weist eine Form ähnlich eines Stromlinienformmodells auf, **dadurch gekennzeichnet, dass** die Form, die ähnlich eines Stromlinienformmodells ist, den Druck in einer Druckverteilung einer Flüssigkeit vorsieht, der größer als der Sättigungsdampfdruck der Flüssigkeit ist, wenn die Ultraschallschwingungen mit einer vorbestimmten Amplitude und Periode von dem Ultraschalloszillator zu dem Behandlungsabschnitt in der Flüssigkeit übertragen werden.

**2.** Ultraschallbehandlungsvorrichtung, **dadurch gekennzeichnet, dass** die Sonde (24) gemäß Anspruch 1 umfasst ist, und die Ultraschallbehandlungsvorrichtung **dadurch gekennzeichnet ist, dass** sie zum Koagulieren und Einschneiden von de, lebendem Gewebe genutzt wird.

**3.** Ultraschallbehandlungsvorrichtung, **dadurch gekennzeichnet, dass** sie umfasst:

die Sonde (24) gemäß Anspruch 1; und einen Kiefer (30), welcher relativ zum Behandlungsabschnitt (26a) geöffnet und geschlossen wird und welcher das Gewebe zusammen mit dem Behandlungsabschnitt (26a) hält.

**4.** Ultraschallbehandlungsvorrichtung, **dadurch gekennzeichnet, dass** sie umfasst:

einen Ultraschalloszillator (18), welcher Ultraschallschwingungen erzeugt; und die Sonde (24) gemäß Anspruch 1.

**5.** Verfahren zum Herstellen einer Sonde für eine Ultraschallbehandlungsvorrichtung, umfassend:

Vorbereiten eines vorherbestimmten Formmodells für zumindest einen Teil eines Behandlungsabschnitts, welcher lebendes Gewebe mit Ultraschallschwingungen behandelt; das Verfahren ist weiter **gekennzeichnet durch** Erhalten einer Druckverteilung durch Strömungsanalyse hinsichtlich Ultraschallschwingungen in einer Flüssigkeit, wobei die Druckverteilung der Flüssigkeit dem Formmodell entspricht; Verändern einer Form eines Kantenabschnitts des Formmodells zu einer Form nahe einer Stromlinienform, so dass der Druck von zumindest einem Teil von Abschnitten, bei denen der Druck gleich oder kleiner als ein Sättigungsdampfdruck der Flüssigkeit in der Druckverteilung ist, größer als der Sättigungsdampfdruck der Flüssigkeit wird; abwechselnd Wiederholen des Erhalten der Druckverteilung der Flüssigkeit und des Verändern der Form des Kantenabschnitts des Formmodells; und nach Beenden der Formveränderung des Kantenabschnitts des Formmodells, nahe der Stromlinienform, Bilden des Behandlungsabschnitts, um eine Form nahe der Stromlinienform des Formmodells aufzuweisen, wenn der Druck von zumindest dem Teil der Abschnitte größer als der Sättigungsdampfdruck der Flüssigkeit wird.

**6.** Verfahren gemäß Anspruch 5, **dadurch gekennzeichnet, dass** das Verändern der Form des Formmodells Verändern der Form des Formmodells beinhaltet, so dass ein Strömungswiderstandskoeffizient reduziert wird.

**7.** Verfahren zum Herstellen einer Ultraschallbehandlungsvorrichtung, **dadurch gekennzeichnet, dass** es das Verfahrens zum Herstellen einer Sonde für eine Ultraschallbehandlungsvorrichtung gemäß Anspruch 5 oder 6 umfasst.

**Revendications**

**1.** Sonde (24) pour un appareil de traitement par ultrasons, la sonde étant connectée à un oscillateur à ultrasons (18) conçu pour générer des oscillations ultrasonores longitudinales dans une direction longitudinale de la sonde (24), la sonde étant conçue pour transmettre les oscillations ultrasonores générées par l'oscillateur à ultrasons (18), et qui comprend une partie de traitement (26a) formée sur la sonde (24) et conçue pour traiter un tissu vivant par les oscillations ultrasonores transmises, la partie de traitement (26a) comprenant une partie de suppression de cavitation (39), et la partie de traitement (26a) ayant une forme proche d'un modèle de forme profilée,

**caractérisée par** la forme proche d'un modèle de forme profilée ayant les pressions dans une distribution de pression d'un liquide supérieures à la pression de vapeur saturante du liquide lorsque les oscillations ultrasonores sont transmises à la partie de traitement à partir de l'oscillateur à ultrasons dans le liquide avec une amplitude et une période prédéterminées.

2. Appareil de traitement par ultrasons **caractérisé en ce qu'**il comprend la sonde (24) selon la revendication 1, et l'appareil de traitement par ultrasons est **caractérisé en ce qu'**il est utilisé pour coaguler et inciser le tissu vivant.

3. Appareil de traitement par ultrasons **caractérisé en ce qu'**il comprend :

   la sonde (24) selon la revendication 1 ; et
   une mâchoire (30) qui est ouverte et fermée par rapport à la partie de traitement (26a), et maintient le tissu en coopération avec la partie de traitement (26a).

4. Appareil de traitement par ultrasons **caractérisé en ce qu'**il comprend :

   un oscillateur à ultrasons (18) qui génère des oscillations ultrasonores ; et
   la sonde (24) selon la revendication 1.

5. Procédé de fabrication d'une sonde pour un appareil de traitement par ultrasons, comprenant :

   la préparation d'un modèle de forme prédéterminé pour au moins une partie d'une partie de traitement qui traite un tissu vivant par des oscillations ultrasonores ;
   le procédé étant en outre **caractérisé par**
   l'obtention, par analyse de fluide concernant des oscillations ultrasonores dans un liquide, d'une distribution de pression du liquide par rapport au modèle de forme ;
   la modification d'une forme d'une partie de bord du modèle de forme en une forme proche d'une forme profilée de telle sorte que la pression d'au moins une partie des parties où la pression est inférieure ou égale à une pression de vapeur saturante du liquide dans la distribution de pression devient supérieure à la pression de vapeur saturante du liquide ;
   la répétition alternativement de l'obtention de la distribution de pression du liquide et de la modification de la forme de la partie de bord du modèle de forme ; et
   le façonnage de la partie de traitement pour avoir une forme proche d'une forme profilée du modèle de forme après avoir terminé la modification de forme proche d'une forme profilée de la partie de bord du modèle de forme lorsque la pression de la partie ou des parties des parties devient supérieure à la pression de vapeur saturante du liquide.

6. Procédé selon la revendication 5, **caractérisé en ce que** la modification de la forme du modèle de forme comprend la modification de la forme du modèle de forme de telle sorte qu'un coefficient de traînée est réduit.

7. Procédé de fabrication d'un appareil de traitement par ultrasons, **caractérisé en ce qu'**il comprend le procédé de fabrication d'une sonde pour un appareil de traitement par ultrasons selon la revendication 5 ou 6.

FIG.1

16

30

39    26a

24

28

FIG. 2

24

C1

B1

26a

FIG. 3A

24

B2

C2

26a

FIG.3B

FIG.4

FIG.5

EP 1 891 905 B1

FIG. 6

Unit [m/s]

FIG.7

FIG. 8

FIG.9

F I G. 10

FIG. 11

F I G. 12

F I G. 13

FIG. 14

FIG. 15

EP 1 891 905 B1

**EP 1 891 905 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2004321606 A
- US 6790216 B
- JP 2002233533 A